# FASCICULE DE BREVET EUROPEEN

(11) **EP 1 438 985 B1**
(45) Date de publication et mention de la délivrance du brevet: **24.02.2010**
(21) Numéro de dépôt: 04290130.6
(22) Date de dépôt: 19.01.2004
(51) Int. Cl.: A61N 1/37

(54) **Dispositif médical implantable actif, notamment stimulateur cardiaque, comprenant des moyens de détermination de la Présence et du type de sonde qui lui est associée**
Implantierbare medizinische Vorrichtung wie ein Herzschrittmacher mit einer Einrichtung zur Feststellung der Präsenz und des Types einer angeordneten Sonde
Implantable medical device e.g. a pacemaker with means for determining the presence and the type of the associated probe

(30) Priorité: 17.01.2003 FR 0300465
(43) Date de publication de la demande: 21.07.2004
(73) Titulaire: ELA MEDICAL, 92541 Montrouge (FR)
(72) Inventeur: Silvestri, Luigi, 10010 Albiano d'Ivrea (TO) (IT); Franceschini, Stefano, 10019 Strambino (TO) (IT)
(74) Mandataire: Dupuis-Latour, Dominique

(56) Documents cités:
- WO-A-02/18009
- US-A- 5 571 156

## Description

L'invention concerne les "dispositifs médicaux implantables actifs" tels que définis par la Directive 90/385/CEE du 20 juin 1990 du Conseil des communautés européennes, notamment les dispositifs stimulateurs cardiaques, défibrillateurs, cardioverteurs et/ou dispositifs "multisite" permettant de délivrer au coeur des impulsions électriques de faible énergie pour le traitement des troubles du rythme cardiaque.

Ces dispositifs comportent un générateur contenant dans un même boîtier les divers circuits électroniques et leur pile d'alimentation. Au moment de l'implantation, ce générateur est relié électriquement et mécaniquement à une sonde pourvue d'électrodes de stimulation intracardiaque permettant de détecter les potentiels de dépolarisation du myocarde et de délivrer à ce dernier les impulsions de stimulation produites par le générateur.

Il est généralement possible de relier à un même générateur deux types de sonde différents, monopolaire ou bipolaire, au choix du chirurgien selon le type de pathologie à traiter. Dans le cas d'une sonde monopolaire (ou "unipolaire"), la détection et la stimulation sont opérées entre l'électrode unique et le boîtier métallique du générateur, tandis que dans une sonde bipolaire, détection et stimulation peuvent effectuées soit en mode différentiel entre deux électrodes de la sonde, soit en mode commun entre le boîtier du générateur et l'une ou l'autre des électrodes.

Bien entendu, de nombreux paramètres internes du générateur doivent être choisis en fonction du type de sonde utilisée, monopolaire ou bipolaire : commutation des bornes devant être utilisées, recueil des signaux de dépolarisation, ajustement des paramètres de stimulation, modification des algorithmes de pilotage du microprocesseur, etc. On comprendra que toute erreur dans la sélection du type de fonctionnement (monopolaire ou bipolaire) peut entraîner des conséquences extrêmement graves : par exemple, si le dispositif est programmé pour une stimulation bipolaire mais qu'il est équipé d'une sonde monopolaire, cette erreur va provoquer une perte de capture et l'application d'une stimulation inappropriée, avec un risque majeur pour le patient.

Par ailleurs, après la fabrication du générateur, il peut s'écouler un laps de temps important entre l'expédition de ce générateur et son implantation dans le corps du patient. Ce délai peut atteindre un an, voire dix-huit mois. Pendant cette période, parfois longue, précédant l'implantation, il est essentiel que l'appareil, dont la pile a été connectée juste avant l'expédition, présente une consommation la plus réduite possible, de manière que la durée de stockage n'ait pas d'incidence notable sur la durée de vie de l'appareil, c'est-à-dire la durée pendant laquelle ce dernier sera fonctionnel après implantation.

Pour ce faire, le dispositif, notamment son microprocesseur, est placé dans un mode de veille à très faible consommation, et il est prévu un mécanisme pour détecter de l'intérieur du dispositif la connexion d'une sonde, afin de détecter ainsi l'implantation et réveiller le circuit du générateur pour le rendre pleinement fonctionnel et initialiser un certain nombre de paramètres, mémoriser des données de départ, etc.

Cette fonction d'activation peut être réalisée de diverses manières.

Ainsi, le US-A-5 350 407 prévoit une goupille d'activation devant être retirée par le chirurgien dans le stimulateur au moment de l'implantation, pour autoriser l'établissement d'une liaison de communication avec un programmateur externe qui enverra un ordre de désactivation au stimulateur.

Les US-A-5 522 856 et US-A-5 370 666 détectent l'insertion d'une sonde par une mesure d'impédance effectuée entre les bornes de la tête de connecteur du dispositif : en l'absence de sonde, cette impédance est extrêmement élevée, mais dès l'insertion d'une sonde la valeur décroît au dessous d'un certain seuil dont le franchissement est détecté pour sortir le stimulateur du mode de veille et le placer dans un mode où il devient totalement fonctionnel.

Le WO-A-02/18009 comprend un circuit "lead status monitor" analysant l'impédance de sonde et les variations de cette impédance, de manière à prendre si nécessaire des décisions appropriées - par exemple une alerte délivrée au patient - en fonction d'un corps de régles prédéterminé.

L'un des buts de l'invention est de proposer un circuit qui puisse, de façon entièrement automatique, détecter le branchement d'une sonde afin de faire basculer le dispositif d'un mode de veille à très faible consommation vers un mode où toutes les fonctionnalités seront activées (avec, corrélativement, un niveau de consommation supérieur).

Comme on le verra, l'invention permet d'atteindre ce but sans aucune intervention du chirurgien, donc sans manipulation ni risque d'oubli (comme dans le cas d'un système nécessitant l'insertion d'une goupille d'activation), sans effectuer de mesure directe et permanente de l'impédance entre les bornes, et sans recours à un programmateur externe.

L'invention a également pour but de proposer un dispositif qui puisse, de manière entièrement autonome, non seulement détecter le branchement d'une sonde, mais également et surtout déterminer le type de sonde utilisée - monopolaire ou bipolaire - et commuter et paramétrer en conséquence les divers circuits et algorithmes du dispositif. Cette fonction, gérée de façon entièrement automatique par le dispositif, permet d'éviter tout risque d'erreur résultant d'un défaut de conformité entre le type de sonde utilisée et le mode de fonctionnement du dispositif (on évite ainsi, par exemple, tout risque de stimulation bipolaire appliquée par erreur à une sonde monopolaire).

Un autre but de l'invention est de proposer un dispositif incorporant une fonction de sécurité permettant de détecter la configuration particulière dans laquelle le stimulateur, après avoir été équipé d'une sonde bipolaire, n'est pas encore introduit dans le site d'implantation (l'incision ou "poche" dans laquelle le chirurgien prévoit de placer le générateur d'impulsions), et n'est pas relié à une électrode ou plaque de référence de potentiel. Or le stimulateur est généralement programmé par défaut en stimulation monopolaire (réglage d'usine). Dans ce cas, il est absolument indispensable que le générateur ne délivre pas d'impulsions de stimulation monopolaire sur la sonde bipolaire, car ces impulsions seraient dangereuses du fait de l'absence de retour de masse, le boîtier du générateur étant électriquement en l'air pendant cette étape ; la sécurité doit être maintenue jusqu'à l'activation fonctionnelle définitive et complète du dispositif.

On verra que le dispositif de l'invention permet de détecter automatiquement cette configuration, pour inhiber la délivrance d'une stimulation bipolaire, inhibition qui sera maintenue tant que la situation persistera et qui sera automatiquement levée dès que la liaison au potentiel de référence sera rétablie par l'insertion du boîtier du générateur dans la poche ou bien par un branchement de celui-ci à une plaque de référence de potentiel, permettant ainsi de refermer électriquement le circuit de masse.

L'activation des fonctionnalités du stimulateur sera ainsi totalement automatique dans le cas d'une sonde bipolaire. Le chirurgien implanteur n'aura pas à utiliser de plaque de référence où à forcer un mode de stimulation bipolaire préalablement à l'implantation du générateur dans la poche ; il lui suffira d'insérer la sonde bipolaire, et la sécurité sera automatiquement assurée pendant la durée nécessaire à l'achèvement de l'implantation.

Un autre but encore de l'invention est de proposer un dispositif permettant d'assurer en outre une surveillance continue, cycle à cycle, de l'intégrité de la sonde en cours de fonctionnement, notamment pour détecter la rupture du conducteur de l'électrode proximale d'une sonde bipolaire, de manière à basculer immédiatement le mode de fonctionnement du dispositif en mode monopolaire, puisque dans ce cas la sonde reste fonctionnelle, mais dans ce dernier mode seulement.

À cet effet, l'invention propose un dispositif du type précité divulgué par le WO-A-02/18009, correspondant au préambule de la revendication 1. De façon caractéristique, l'invention prévoit d'opérer une discrimination sur la largeur du signal impulsionnel recueilli sur les bornes du générateur lors de l'application d'impulsions de stimulation monopolaires ou bipolaires aux électrodes de la sonde. Les sous-revendications explicitent divers modes de mise en oeuvre avantageux.

On va maintenant décrire un exemple de mise en oeuvre de l'invention, en référence aux dessins annexés.

La figure 1 est une représentation schématique du dispositif selon l'invention, avec un générateur équipé d'une sonde bipolaire.

Les figures 2 à 5 représentent, pour quatre situations différentes, d'une part (à gauche) le circuit électrique équivalent vu des bornes du dispositif et d'autre part (à droite) les formes des impulsions susceptibles d'être recueillies sur les diverses bornes du dispositif à l'application d'une impulsion de stimulation, dans le cas où la liaison au potentiel de référence a été volontairement déconnectée.

Les figures 6 à 8 sont homologues des figures 2 à 5, dans le cas où une liaison au potentiel de référence est établie.

La figure 9 est un organigramme montrant les différentes étapes de l'algorithme de détection de la présence d'une sonde et du type de sonde, ainsi que des actions prises en conséquence.

La figure 10 est un organigramme montrant la manière dont il est possible de détecter, en cours de fonctionnement, une perte d'intégrité d'une sonde bipolaire.

La figure 11 est un organigramme des étapes montrant les différentes étapes de l'algorithme permettant, d'une part, d'augmenter la durée de vie du dispositif par mise en veille avant l'implantation et, d'autre part, la détection de la configuration où le boîtier, après avoir été pourvu d'une sonde bipolaire, n'a pas encore été introduit dans la poche par le chirurgien.

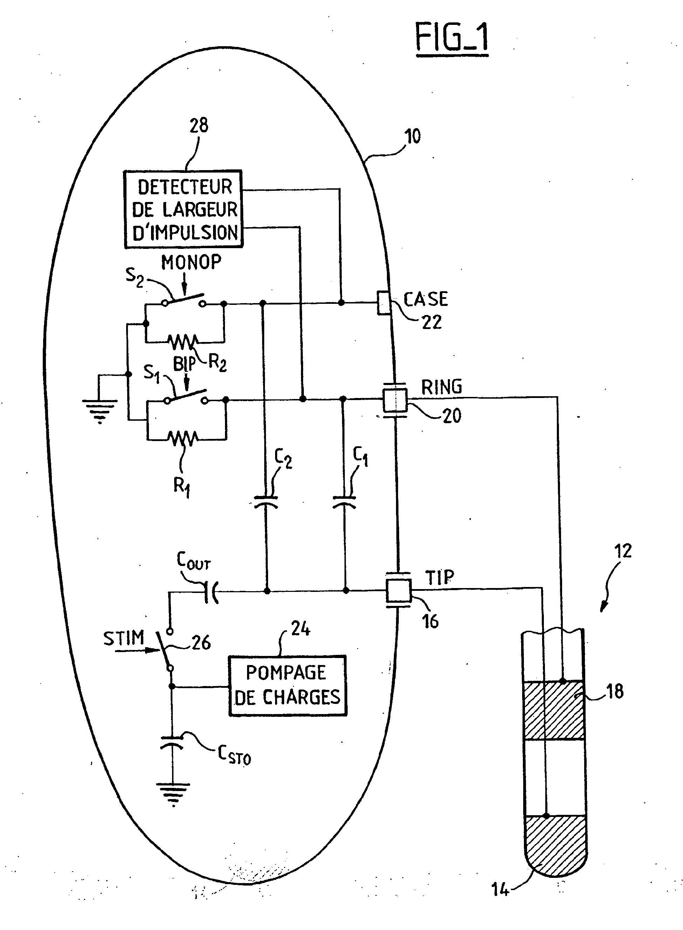

La figure 1 représente, de façon schématique, un dispositif stimulateur cardiaque (ou un dispositif incluant des fonctions de stimulation cardiaque), comprenant essentiellement un générateur 10 et une sonde de stimulation 12.

Sur la figure 1, la sonde illustrée est une sonde bipolaire, comprenant donc deux électrodes :
- une électrode distale 14, en bout de sonde, reliée à une borne 16 du générateur, borne généralement désignée "TIP" ;
- une électrode proximale 18, de forme annulaire, reliée à une autre borne 20 du générateur, généralement désignée "RING".

Par ailleurs, le boîtier métallique du générateur est relié en interne à une borne 22, généralement désignée "CASE", et utilisée comme référence de potentiel par rapport aux électrodes RING et TIP pour le recueil des potentiels de dépolarisation et/ou l'application d'impulsions de stimulation.

Le générateur comporte un circuit de pompage de charges 24 assurant la charge d'un condensateur de forte capacité C_{STO}. Ce condensateur une fois chargé permet de délivrer l'impulsion de stimulation par fermeture de l'interrupteur 26 piloté par une commande STIM délivrée par le microcontrôleur, qui a pour effet de transférer l'énergie accumulée dans le condensateur C_{STO} vers la borne TIP 16, via un condensateur de découplage C_{OUT}.

Dans le cas d'une sonde bipolaire (cas illustré sur la figure 1), c'est la borne RING 20 qui constitue la référence électrique, et qui est donc reliée à la masse interne du générateur par fermeture d'un interrupteur S₁ piloté par un signal BIP produit par le microcontrôleur. Lorsque S₁ est ouvert, une résistance R₁ est intercalée entre la masse et l'électrode 20 ; par ailleurs, un condensateur C₁ entre les bornes 16 et 20 assure une protection contre les diverses interférences électromagnétiques.

En mode monopolaire, c'est la borne CASE 22 qui constitue la référence électrique, et qui est reliée à la masse interne du générateur par fermeture d'un interrupteur S₂ piloté par un signal MONOP produit par le microcontrôleur. Lorsque S₂ est ouvert, une résistance R₂ est intercalée entre la masse et l'électrode 22 ; par ailleurs, un condensateur C₂ entre les bornes 16 et 22 assure une protection contre les diverses interférences électromagnétiques.

Essentiellement, le mécanisme de détection d'une sonde, et du type de sonde, selon l'invention consiste à appliquer des impulsions de stimulation bipolaires ou monopolaires, ou avec déconnexion de la liaison au potentiel de référence, et à observer les signaux produits sur les électrodes CASE et RING, signaux qui seront notablement différents selon que :
- le générateur n'est pas implanté et n'est pas encore pourvu de sonde,
- il est pourvu d'une sonde bipolaire mais pas encore implanté, ou
- il est implanté et pourvu d'une sonde bipolaire, ou
- il est implanté et pourvu d'une sonde monopolaire.

Comme on va le décrire, cette discrimination peut être réalisée par une analyse de largeur d'impulsions et une comparaison de cette largeur à différents seuils.

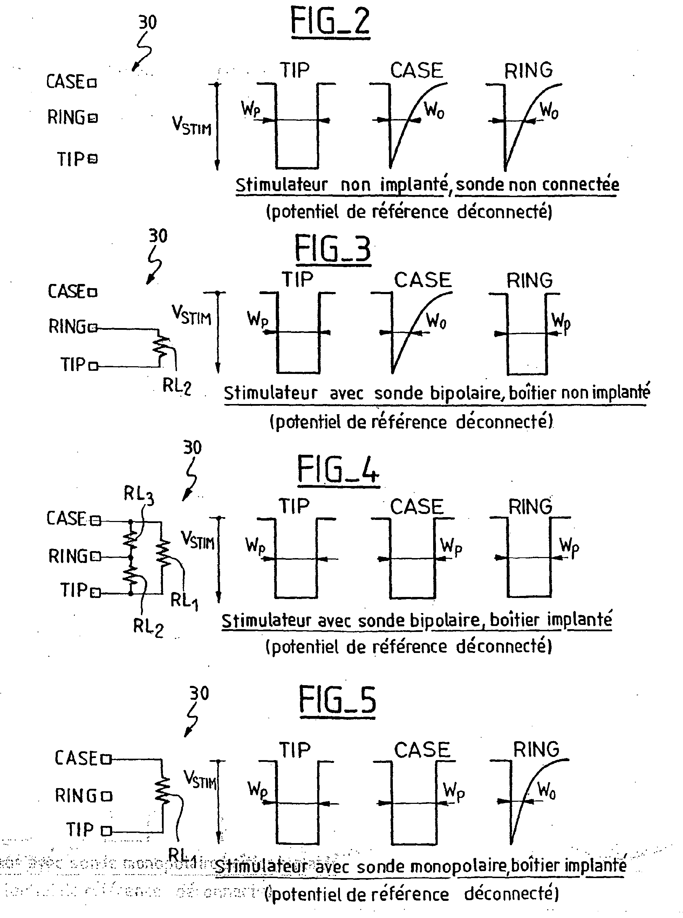

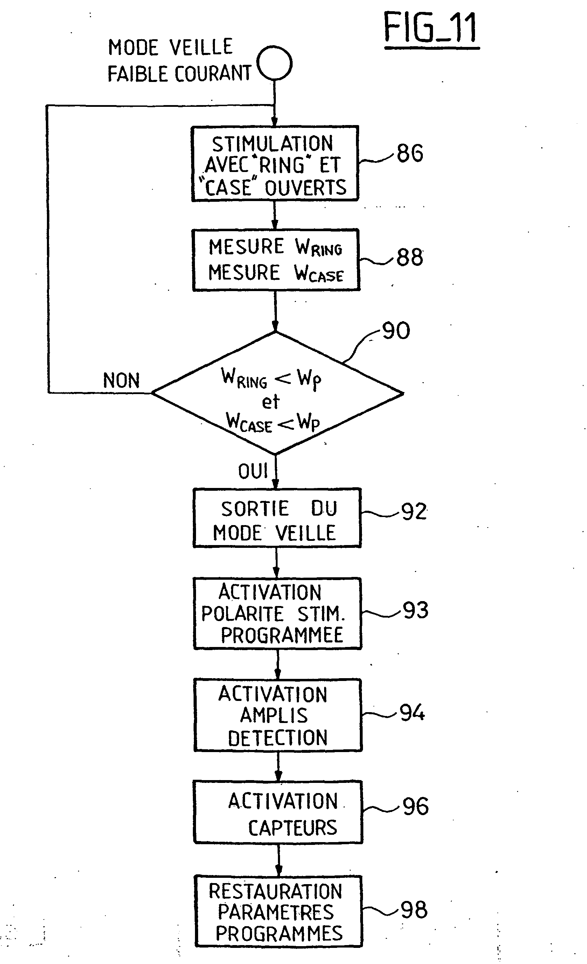

Une première possibilité, illustrée par les figures 2 à 5, consiste à délivrer une impulsion de stimulation avec les deux liaisons au potentiel de référence déconnectées (c'est-à-dire S₁ et S₂ ouverts), et à observer les formes d'onde recueillies sur les électrodes RING et CASE. Cette possibilité, bien qu'elle nécessite l'analyse du signal sur deux électrodes et la délivrance d'un impulsion de stimulation qui n'est pas capturée par le coeur, présente l'avantage d'autoriser la détection d'un dispositif partiellement implanté, avec une sonde bipolaire reliée au générateur mais celui-ci étant situé hors de la poche. Cette faculté peut être en particulier utilisée, comme on l'exposera plus loin, pour "réveiller" le générateur en le faisant sortir d'un mode à basse consommation et en exécutant un certain nombre d'étapes d'initialisation, comme on l'exposera plus bas, en référence notamment à l'organigramme de la figure 11.

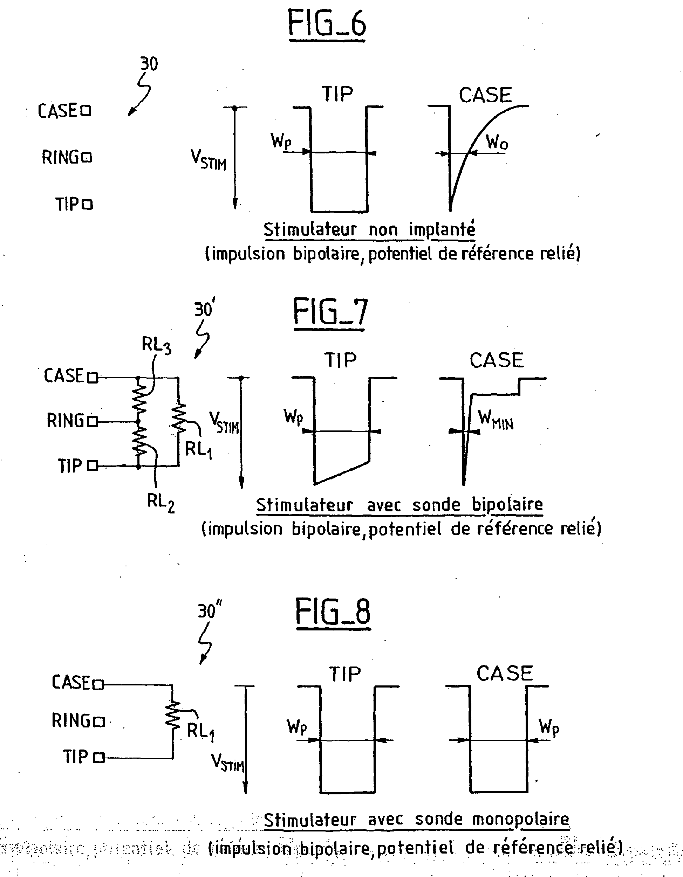

L'autre possibilité, illustrée par les figures 6 à 8, consiste à délivrer une impulsion de stimulation avec les deux liaisons au potentiel de référence établies (c'est-à-dire S₁ et S₂ fermés). Dans la suite de la description, on envisagera dans ce cas que l'impulsion de stimulation appliquée est une impulsion bipolaire, c'est-à-dire appliquée entre les bornes TIP et RING. Mais l'invention pourrait être mise en oeuvre de façon comparable en appliquant une impulsion de stimulation monopolaire (donc entre les électrodes CASE et TIP) et en observant la forme de l'impulsion recueillie sur l'électrode RING. Cette variante, bien que moins avantageuse, doit néanmoins être comprise comme entrant dans le cadre de la présente invention.

La détection de la présence d'une sonde, du type de sonde et de l'implantation effective est opérée en mesurant au moyen d'un circuit détecteur de largeur d'impulsion 28 la largeur des impulsions recueillies sur les bornes CASE 22 et RING 20, de la manière que l'on va maintenant exposer.

On va maintenant décrire, en référence aux figures 2 à 5, la manière dont l'invention permet de détecter le raccordement d'une sonde et l'implantation du générateur.

Dans l'état initial, à l'expédition du dispositif, où aucune sonde n'est branchée, la configuration extérieure 30 (figure 2) des électrodes CASE, RING et TIP vue par le dispositif correspond à une impédance très élevée, théoriquement infinie, entre deux quelconques de ces trois bornes. Dans ces conditions, une impulsion de stimulation appliquée par le dispositif avec les deux liaisons au potentiel de référence déconnectées (c'est-à-dire S₁ et S₂ ouverts) a la forme illustrée figure 2, à savoir une forme de créneau rectangulaire de hauteur V_{STIM} et de largeur W_{P}. Toujours dans cette configuration, les bornes CASE et RING sont couplées à la borne TIP par l'intermédiaire des condensateurs de protection respectifs C₂ et C₁. Le circuit 28 va donc voir sur ces bornes CASE et RING des impulsions telles qu'illustrées sur la figure 2, avec un flanc abrupt puis un retour au niveau initial selon une constante de temps définie par R₁ et C₁ ou R₂ et C₂, la durée W_{O} à mi-hauteur des impulsions ainsi recueillies étant de l'ordre de 200 µs.

La figure 3 illustre le cas où le stimulateur a été équipé d'une sonde bipolaire, mais où le boîtier du générateur n'a pas encore été implanté dans la poche, ni relié à une électrode ou plaque de référence de potentiel. Il s'agit d'une configuration particulière qui doit être détectée pour empêcher une stimulation monopolaire sur sonde bipolaire. Dans ce cas, le réseau d'impédances 30, vu de l'intérieur du stimulateur, ne comporte qu'une impédance RL₂ entre les deux électrodes RING et TIP, puisque l'électrode CASE est électriquement en l'air. L'impulsion de stimulation appliquée sur la borne TIP a toujours la forme d'un créneau et l'impulsion recueillie sur la borne CASE présente la même allure que précédemment, du fait du seul couplage interne par le condensateur de protection C₂, avec un flanc abrupt puis un retour progressif au niveau initial. En revanche, l'impulsion recueillie sur la borne RING présente la forme d'un créneau semblable à l'impulsion de stimulation délivrée sur la borne TIP, du fait du couplage externe par l'impédance RL₂.

La figure 4 illustre le cas où le stimulateur a été équipé d'une sonde bipolaire, avec le boîtier du générateur implanté dans la poche ou bien relié à une électrode de référence. Dans ce cas, le réseau d'impédances 30 correspondant à cette sonde, vu de l'intérieur du stimulateur, comprend des impédances RL₁, RL₂ et RL₃ entre les trois électrodes TIP, CASE et RING prises deux à deux. Les impulsions recueillies sur les bornes CASE et RING présentent alors toutes deux la forme d'un créneau semblable à l'impulsion de stimulation délivrée sur la borne TIP, du fait du couplage externe avec cette dernière par les impédance RL₁, RL₂ et RL₃.

Le dernier cas, illustré figure 5, est celui où le stimulateur est équipé d'une sonde monopolaire, avec le boîtier du générateur implanté dans la poche ou bien relié à une électrode de référence (on notera qu'avec une sonde monopolaire, si le boîtier n'a pas encore été implanté, ni relié à une électrode de référence, la situation est identique à celle de la figure 2 ; le stimulateur étant par défaut en stimulation monopolaire (réglage d'usine), la situation ne présente pas de risque et aucune mesure de sécurité particulière n'est à prendre préalablement à l'activation fonctionnelle complète du dispositif). La configuration d'impédance 30 est celle illustrée figure 5, à savoir une unique impédance RL₁ entre CASE et TIP, avec une impédance très élevée, théoriquement infinie, entre RING et CASE ou RING et TIP. L'impulsion recueillie sur la borne CASE présente la forme d'un créneau semblable à l'impulsion de stimulation délivrée sur la borne TIP, du fait du couplage externe par l'impédance RL₁. En revanche, l'impulsion recueillie sur la borne RING présente un flanc abrupt puis un retour progressif au niveau initial, du fait du couplage interne par le condensateur de protection C₁.

Ainsi, la simple analyse des impulsions sur les bornes CASE 22 et RING 20 par le circuit détecteur 28 permet de discriminer entre un générateur non implanté (figure 2) et un générateur partiellement implanté (Figures 3 à 5). Dans le cas du générateur non implanté la largeur du signal détecté aussi bien sur les bornes CASE que RING est W_{O}. Dès qu'une quelconque sonde est connectée, la largeur sur CASE et/ou RING devient W_{P}.

Cette discrimination permet de détecter immédiatement, c'est-à-dire dans l'intervalle d'un battement cardiaque, le raccordement, même partiel, d'une sonde bipolaire ou monopolaire, que le générateur soit ou non placé dans la poche.

Cette détection est notamment utilisée pour "réveiller" immédiatement le générateur et le sortir d'un mode à basse consommation dans lequel il se trouvait et le commuter dans un mode où il sera pleinement fonctionnel, avec en particulier une stimulation de sécurité assurant la capture de l'impulsion de stimulation pendant la durée de la procédure d'implantation.

La mise en oeuvre de ces fonctions sera décrite plus en détail par la suite, en référence à l'organigramme de la figure 11.

On va maintenant décrire, en référence aux figures 6 à 8, la manière dont l'invention permet de détecter que le dispositif a été correctement implanté et que la phase d'implantation est achevée.

Pour ce faire, on applique une impulsion bipolaire (par exemple) avec les deux liaisons au potentiel de référence établies (c'est-à-dire S₁ et S₂ fermés) et on analyse la forme du signal recueilli sur les bornes TIP et CASE.

La figure 6 montre la forme de l'impulsion recueillie sur la borne CASE pour une impulsion bipolaire appliquée entre les bornes TIP et RING, lorsque le générateur n'est pas implanté : l'impulsion observée sur la borne CASE présente un front très raide suivi d'une décroissance exponentielle relativement lente, définie par la constante de temps des composants internes

R₁ et C₁, la durée W_{O} de l'impulsion à mi-niveau étant d'environ 200 µs.

La figure 7 montre la forme de l'impulsion recueillie sur la borne CASE lorsque l'impulsion bipolaire est appliquée avec le générateur correctement implanté et pourvu d'une sonde bipolaire : dans un tel cas, le circuit du générateur voit trois impédances RL₁, RL₂ et RL₃ entre les trois électrodes respectives TIP, CASE et RING de la sonde. La forme de l'impulsion observée sur la borne CASE lorsqu'une impulsion de stimulation est appliquée entre les bornes TIP et RING chute à V_{STIM} et remonte rapidement, avec un constante de temps RL₁/(RL₂+RL₃)*C₁. Pour une impédance maximale de 2 kilohms, la durée maximale de l'impulsion est inférieure à 20 µs, valeur définissant un seuil bas W_{MIN}.

La figure 8 montre la forme de l'impulsion recueillie sur la borne CASE lorsque l'impulsion bipolaire est appliquée entre les bornes TIP et CASE avec le générateur correctement implanté et pourvu d'une sonde monopolaire : dans un tel cas, l'impédance entre TIP et CASE est seulement celle des tissus, tandis que celle entre TIP et RING, ou entre RING et CASE, est très élevée, théoriquement infinie. La forme du signal observé sur la borne CASE est de ce fait pratiquement la même que celle de l'impulsion détectée sur la borne TIP, en raison de la faible impédance RL₁ couplant les bornes CASE et TIP.

Ainsi, la simple analyse par le circuit détecteur 28 de la largeur des impulsion sur les bornes CASE 22 et RING 20 (largeur prise par exemple à mi-hauteur) permet de déterminer la configuration d'impédances et donc les trois cas de figure que l'on vient de décrire, permettant de déterminer la présence d'une sonde, le type de sonde et l'intégrité du circuit de retour de masse résultant de l'implantation effective dans la poche. Le circuit 28 est un circuit dont les seuils de détection ont été programmés de manière appropriée pour discriminer de façon non ambiguë les trois cas de figures possibles que l'on vient d'exposer.

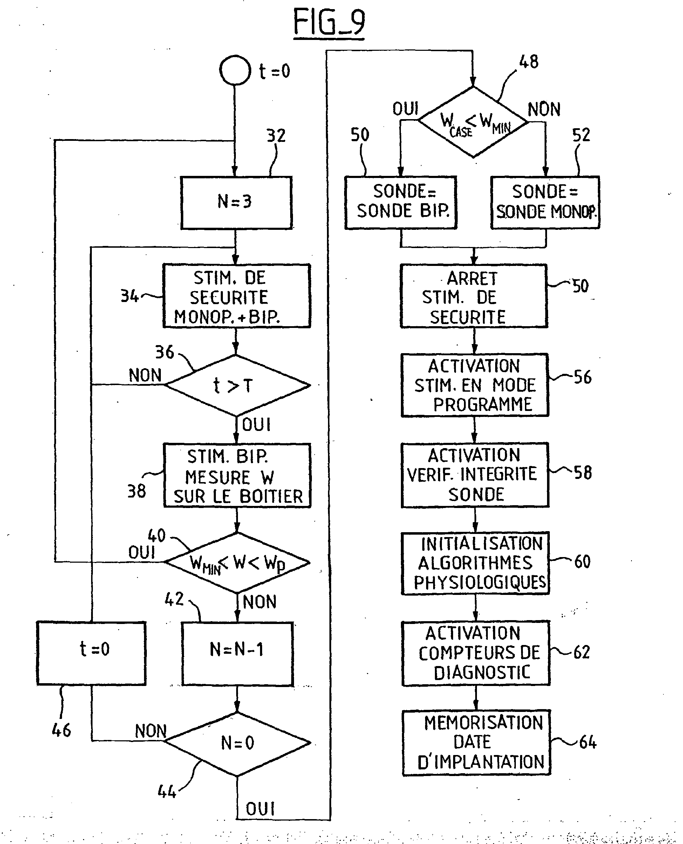

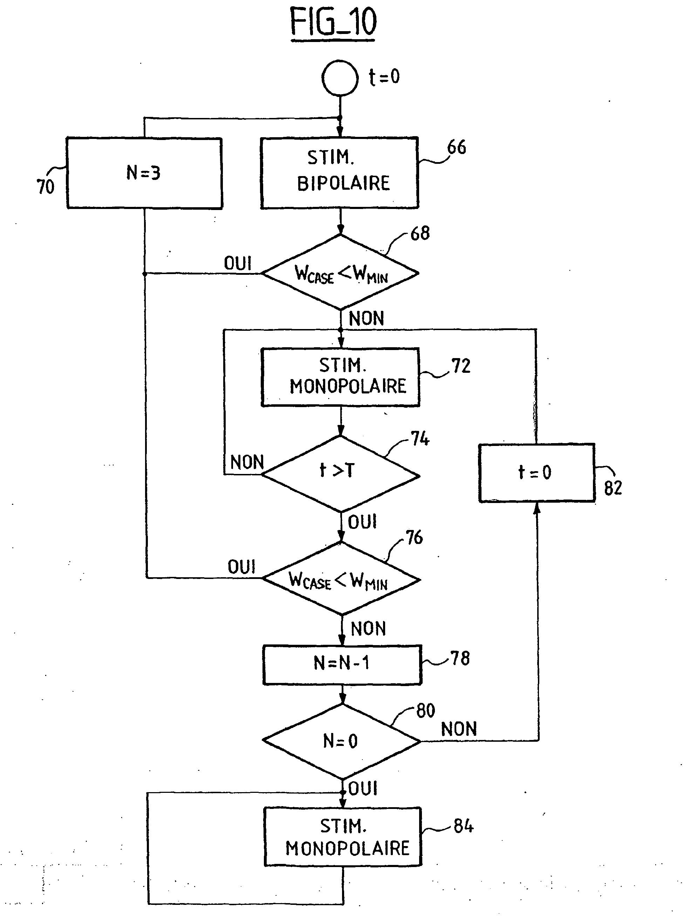

On va maintenant exposer le fonctionnement du dispositif de l'invention, en référence aux organigrammes des figures 9 à 11. Dans la description relative aux organigrammes des figures 9 et 10, pour la clarté de l'exposé on fera abstraction de la protection spécifique correspondant au cas particulier où le stimulateur a été équipé d'une sonde bipolaire, mais où le boîtier du générateur n'a pas encore été implanté dans la poche, ni relié à une électrode ou plaque de référence de potentiel ; cette situation particulière sera traitée en référence à l'organigramme de la figure 11.

La figure 9 est un organigramme montrant les diverses étapes de l'algorithme permettant de détecter l'implantation du dispositif et paramétrer celui-ci en conséquence.

Après mise en place de la pile, au moment de l'expédition (t = 0) et après initialisation d'un compteur N (étape 32), le générateur opère dans un mode de stimulation dit "de sécurité" avec délivrance simultanée d'impulsions de stimulation bipolaires et monopolaires (étape 34), à une fréquence relativement faible, de l'ordre de 70 cpm.

De la sorte, lorsqu'une sonde est connectée au générateur par le chirurgien juste avant l'implantation proprement dite, les impulsions seront déjà délivrées sur l'électrode (ou les électrodes) de la sonde, que cette dernière soit monopolaire ou bipolaire. Après écoulement d'une période de temps prédéterminée T (étape 36), le générateur détecte la largeur W de l'impulsion captée sur la borne CASE du générateur opérant en mode de stimulation bipolaire (étape 38). Si la largeur de cette impulsion est supérieure à une valeur de seuil prédéterminée W_{MIN} et inférieure à la valeur W_{P} de l'impulsion de stimulation (étape 40), ceci signifie que le dispositif n'est pas encore implanté, et ce dernier retourne alors au mode de stimulation de sécurité de l'étape 34.

Dans le cas contraire (étape 42), le compteur N est décrémenté d'une unité et, s'il n'est pas nul (étape 44), le comptage de la période T est réinitialisé (étape 46) et le dispositif retourne à l'étape 34 du mode de stimulation de sécurité.

Dans le contraire, ceci signifie que le test de l'étape 40 a détecté trois fois de suite sur la borne CASE une impulsion de largeur inférieure à W_{MIN} ou égale à W_{P}, c'est-à-dire a détecté la présence d'une sonde, donc le fait que le dispositif a été définitivement implanté (par précaution, la période T est choisie de manière que 3.T corresponde à une durée supérieure à la durée moyenne d'une implantation, c'est-à-dire 3.T > 20 mn environ).

Une fois la présence d'une sonde détectée, le stimulateur détermine, en fonction de la largeur d'impulsion sur la borne CASE, s'il s'agit d'une sonde bipolaire ou monopolaire (étapes 48, 50 et 52). si la largeur de l'impulsion est inférieure à W_{MIN}, la sonde implantée est une sonde bipolaire, tandis que si cette largeur est supérieure ou égale à W_{P}, il s'agit d'une sonde monopolaire.

La stimulation de sécurité est alors arrêtée (étape 54) et la stimulation en mode programmé est activée (étape 56).

À ce stade est également activé un algorithme permettant, selon l'invention, une vérification continuelle de l'intégrité de la sonde (étape 58, qui sera décrite plus en détail en référence à la figure 10).

Enfin, les algorithmes physiologiques sont initialisés (étape 60), les compteurs de diagnostic sont remis à zéro et activés (étape 62) et, si le dispositif est pourvu d'une horloge temps réel, la date d'implantation est inscrite en mémoire (étape 64).

La figure 10 illustre la manière dont le procédé de l'invention permet la surveillance en continu de l'intégrité d'une sonde bipolaire après implantation de celle-ci. Ce procédé permet notamment de détecter une rupture du conducteur de l'électrode proximale 18, qui aurait pour conséquence une perte de capture susceptible d'entraîner des risques majeurs pour le patient. Cette rupture a pour effet de modifier l'impédance du circuit équivalent 30 de telle sorte que l'impulsion reçue sur l'électrode CASE va présenter la forme illustrée, avec une largeur W_{P}.

Dès lors, si la stimulation bipolaire est programmée (étape 66) et que la largeur W de l'impulsion recueillie sur la borne CASE est inférieur au seuil W_{MIN} (étape 68), ceci signifie que la sonde est intègre (voir plus bas). La stimulation bipolaire est alors poursuivie (retour à l'étape 66, avec forçage à une valeur prédéterminée d'un compteur N, par exemple N = 3).

Dans le cas contraire, ceci signifie que la sonde bipolaire se comporte comme une sonde monopolaire, ce qui est révélé par un raccourcissement de l'impulsion recueillie sur la borne CASE, jusqu'à une largeur sensiblement égale à W_{P}. Le dispositif bascule alors en mode de stimulation monopolaire (étape 72) par sécurité. Cette condition est temporaire, jusqu'à être confirmée par trois tests concordants successifs au cours d'un intervalle de temps donné T (étapes 72 à 82). Si le défaut est ainsi confirmé, alors le stimulateur bascule définitivement en stimulation monopolaire (étape 84).

On notera que ce procédé peut être également utilisé pour éviter que, par erreur, un praticien ne reprogramme une stimulation bipolaire sur un dispositif qui avait été implanté avec une sonde monopolaire, situation qui aurait les mêmes conséquences que la rupture du conducteur d'électrode proximale d'une sonde bipolaire.

Ainsi, après que le praticien ait programmé une stimulation en mode bipolaire, le dispositif produit une première stimulation bipolaire et détecte sur la borne CASE une impulsion d'une certaine largeur W :
- si cette largeur W est inférieure à W_{MIN}, la configuration est conforme et la modification de programmation est acceptée ;
- dans le cas contraire, alors la sonde implantée est une sonde monopolaire, incompatible avec une stimulation bipolaire, et le changement de mode de programmation est refusé par le dispositif.

La figure 11 illustre la manière dont le procédé de l'invention peut être utilisé pour améliorer la durée de vie du dispositif, et pour prendre en compte le cas particulier où le stimulateur a été équipé d'une sonde bipolaire, mais où le boîtier du générateur n'a pas encore été implanté dans la poche, ni relié à une électrode ou plaque de référence de potentiel.

On sait que de nombreuses fonctions du dispositif entraînent une consommation de courant élevée, mais ne sont pas nécessaires avant l'implantation. La durée de vie de la pile, et donc la date de péremption du dispositif avant implantation, peuvent être améliorés si ces fonctions sont désactivées à la livraison du dispositif et sont automatiquement activées lorsque la connexion d'une sonde est détectée.

Comme illustré sur la figure 11, à la livraison le générateur est en mode de veille, consommant un faible courant. Notamment, les circuits analogiques (amplificateurs de détection, circuits de polarisation des capteurs et circuits analogiques de traitement des signaux) sont désactivés et les fonctions du microcontrôleur sont réduites au minimum, avec une simple stimulation à un rythme lent, par exemple 70 cpm sur l'oreillette et le ventricule, stimulation suffisante pour permette la détection d'une sonde (étapes 86 à 90).

Dés que la présence d'une sonde, auriculaire ou ventriculaire, est détectée, et que l'implantation effective est confirmée ou que le boîtier est relié à une électrode de référence (étape 90, où l'on teste que la largeur de l'impulsion sur la borne CASE est sensiblement égale à la largeur W_{P} de l'impulsion de stimulation), alors le dispositif bascule son mode de fonctionnement en sortant du mode de veille à faible consommation (étape 92), en activant le mode de stimulation programmé, monopolaire ou bipolaire (étape 93), en déclenchant l'activation des amplificateurs de détection et autres circuits analogiques (étape 94), en activant les capteurs physiologiques ou d'activité (étape 96) et en restaurant les paramètres programmés (étape 98).

## Revendications

1. Un dispositif médical implantable actif, notamment un dispositif stimulateur cardiaque, défibrillateur, cardioverteur et/ou dispositif "multisite" permettant de délivrer au coeur des impulsions électriques de faible énergie pour le traitement des troubles du rythme cardiaque,
ce dispositif comprenant un boîtier métallique (10) contenant un générateur, apte à produire des impulsions de stimulation en mode monopolaire ou en mode bipolaire, relié à une tête de connecteur pourvue d'au moins deux bornes (RING, TIP) aptes à être reliées à des électrodes d'une sonde (12) de détection et de stimulation reliée au connecteur, cette sonde pouvant être une sonde monopolaire ou une sonde bipolaire,
dispositif dans lequel le générateur inclut des moyens de détection de la présence d'une sonde reliée au connecteur, comprenant:
- des moyens (24, 26) pour produire des impulsions de stimulation monopolaires ou bipolaires, sélectivement avec ou sans déconnexion de la liaison (S₁, S₂) au potentiel de référence ;
- des moyens pour recueillir au moins un signal impulsionnel produit par la variation de potentiel induite sur l'une et/ou l'autre des bornes (RING, TIP) et/ou sur le boîtier métallique (CASE) par l'application desdites impulsions de stimulation ;
- des moyens discriminateurs (28), propres à analyser une caractéristique de forme dudit au moins un signal impulsionnel et à délivrer un indicateur représentatif de la présence ou de l'absence d'une sonde ; et
- des moyens de commande, pour modifier au moins un paramètre de fonctionnement du dispositif sélectivement en fonction de l'indicateur délivré par les moyens discriminateurs,
**caractérisé en ce que**
ladite caractéristique de forme est la largeur dudit au moins un signal impulsionnel.

2. Le dispositif de la revendication 1, dans lequel les moyens pour produire des impulsions de stimulation sont des moyens aptes à appliquer une impulsion de stimulation avec déconnexion de la liaison au potentiel de référence, et les moyens discriminateurs sont des moyens aptes à comparer à un seuil donné (W_{P}) la largeur du signal impulsionnel recueilli sur la borne de l'électrode proximale (RING) et sur le boîtier (CASE).

3. Le dispositif de la revendication 2, dans lequel les moyens discriminateurs sont des moyens aptes à délivrer un indicateur représentatif de l'absence d'une sonde (figure 2) ou de la non-implantation du boîtier après connexion d'une sonde (figure 3) lorsque la largeur du signal impulsionnel sur le boîtier (CASE) est inférieure audit seuil (W_{P}).

4. Le dispositif de la revendication 2, dans lequel les moyens discriminateurs sont des moyens aptes à délivrer un indicateur représentatif de la présence d'une sonde monopolaire implantée (figure 5) lorsque la largeur du signal impulsionnel recueilli sur le boîtier (CASE) est supérieure ou égale audit seuil (W_{P}) et la largeur du signal impulsionnel recueilli sur la borne de l'électrode proximale (RING) est inférieure audit seuil (Wp).

5. Le dispositif de la revendication 2, dans lequel les moyens discriminateurs sont des moyens aptes à délivrer un indicateur représentatif de la présence d'une sonde bipolaire implantée (figure 4) lorsque la largeur du signal impulsionnel recueilli sur le boîtier (CASE) et sur la borne proximale (RING) est supérieure ou égale audit seuil (W_{P}).

6. Le dispositif de la revendication 1, dans lequel les moyens pour produire des impulsions de stimulation sont des moyens aptes à appliquer une impulsion de stimulation bipolaire sans déconnexion de la liaison au potentiel de référence, et les moyens discriminateurs sont des moyens aptes à comparer la largeur du signal impulsionnel recueilli sur le boîtier (CASE) à un seuil bas (W_{MIN}) et à un seuil haut (W_{P}).

7. Le dispositif de la revendication 6, dans lequel les moyens discriminateurs sont des moyens aptes à délivrer un indicateur représentatif de la présence d'une sonde bipolaire (figure 7) lorsque la largeur du signal impulsionnel sur le boîtier (CASE) est inférieure audit seuil bas (W_{MIN}).

8. Le dispositif de la revendication 6, dans lequel les moyens discriminateurs sont des moyens aptes à délivrer un indicateur représentatif de l'absence d'une sonde (figure 6) lorsque la largeur du signal impulsionnel sur le boîtier (CASE) est comprise entre ledit seuil bas (W_{MIN}) et ledit seuil haut (W_{P}).

9. Le dispositif de la revendication 6, dans lequel les moyens discriminateurs sont des moyens aptes à délivrer un indicateur représentatif de la présence d'une sonde monopolaire (figure 8) lorsque la largeur du signal impulsionnel sur le boîtier (CASE) est égale audit seuil haut (W_{P}).

10. Le dispositif de la revendication 1, dans lequel les moyens pour produire des impulsions de stimulation sont des moyens aptes à appliquer une impulsion de stimulation monopolaire sans déconnexion de la liaison au potentiel de référence, et les moyens discriminateurs sont des moyens aptes à comparer la largeur du signal impulsionnel recueilli sur la borne de l'électrode proximale (RING) à un seuil bas (W_{MIN}) et à un seuil haut (W_{P}).

11. Le dispositif de la revendication 10, dans lequel les moyens discriminateurs sont des moyens aptes à délivrer un indicateur représentatif de l'absence d'une sonde (figure 6) lorsque la largeur du signal impulsionnel sur la borne de l'électrode proximale (RING) est comprise entre ledit seuil bas (W_{MIN}) et ledit seuil haut (W_{P}).

12. Le dispositif de la revendication 10, dans lequel les moyens discriminateurs sont des moyens aptes à délivrer un indicateur représentatif de la présence d'une sonde bipolaire lorsque la largeur du signal impulsionnel sur la borne de l'électrode proximale (RING) est inférieure audit seuil bas (W_{MIN}).

13. Le dispositif de la revendication 10, dans lequel les moyens discriminateurs sont des moyens aptes à délivrer un indicateur représentatif de la présence d'une sonde monopolaire lorsque la largeur du signal impulsionnel sur la borne de l'électrode proximale (RING) est égale audit seuil haut (W_{P}).

14. Le dispositif de la revendication 1, dans lequel les moyens pour produire des impulsions de stimulation sont des moyens aptes à appliquer une impulsion de stimulation bipolaire entre la borne de l'électrode proximale (RING) et la borne de l'électrode distale (TIP), sans déconnexion de la liaison au potentiel de référence, et les moyens discriminateurs sont des moyens aptes à comparer la largeur du signal impulsionnel recueilli sur le boîtier (CASE) à un seuil haut (W_{P}) et à un seuil bas (W_{MIN}).

15. Le dispositif de la revendication 14, dans lequel les moyens discriminateurs sont des moyens aptes à délivrer un indicateur représentatif de l'absence d'une sonde ou de la non-implantation du boîtier après connexion d'une sonde (figure 6) lorsque la largeur du signal impulsionnel sur le boîtier (CASE) est comprise entre ces deux seuils.

16. Le dispositif de la revendication 15, dans lequel les moyens discriminateurs sont des moyens aptes à délivrer un indicateur représentatif de la présence d'une sonde bipolaire (figure 7) lorsque la largeur du signal impulsionnel sur le boîtier (CASE) est inférieure audit seuil bas (W_{MIN}).

17. Le dispositif de la revendication 15, dans lequel les moyens discriminateurs sont des moyens aptes à délivrer un indicateur représentatif de la présence d'une sonde monopolaire (figure 8) lorsque la largeur du signal impulsionnel sur le boîtier (CASE) est égale audit seuil haut (W_{P}).

18. Le dispositif de la revendication 1, dans lequel, à partir d'un état initial du dispositif avant implantation où celui-ci opère temporairement dans un mode à fonctionnalités réduites, les moyens de commande sont des moyens aptes à faire passer, sur délivrance d'un indicateur représentatif de la présence d'une sonde, le dispositif dans un mode pleinement fonctionnel.

19. Le dispositif de la revendication 18, dans lequel les moyens de commande sont des moyens aptes, sur délivrance d'un indicateur représentatif de la présence d'une sonde, à exécuter en outre au moins une action parmi : activation des circuits analogiques de détection du dispositif ; activation des capteurs physiologiques ou d'activité ; programmation du type de stimulation, monopolaire ou bipolaire, en fonction de l'indicateur délivré ; initialisation des algorithmes de fonctionnement du dispositif ; remise à zéro et activation des compteurs de diagnostic ; inscription en mémoire de la date d'implantation.

20. Le dispositif de la revendication 1, dans lequel il est en outre prévu des moyens de sécurité aptes, lorsque le dispositif opère en stimulation bipolaire sur une sonde bipolaire et en cas de délivrance d'un indicateur non représentatif de la présence d'une sonde bipolaire, à faire passer le dispositif dans un mode de sécurité avec stimulation monopolaire.

21. Le dispositif de la revendication 1, dans lequel lesdits moyens de sécurité sont des moyens aptes, lorsque le dispositif reçoit d'un programmateur externe une instruction de paramétrage en mode de stimulation bipolaire, à n'autoriser l'activation du mode de stimulation bipolaire qu'à délivrance par moyens discriminateurs d'un indicateur représentatif de la présence d'une sonde bipolaire.

## Claims

1. An active implantable medical device, in particular a cardiac pacemaker, a defibrillator, a cardioverter and/or a "multisite" device adapted to deliver to the heart low energy electrical pulses for the treatment of heart rhythm disorders,
wherein said device comprises a metallic housing (10) containing a generator adapted to produce stimulation pulses in a monopolar mode or in a bipolar mode, connected to a connector head provided with at least two terminals (RING, TIP) adapted to be connected to the electrodes of a detection and stimulation probe (12) connected to the connector, wherein said probe can be a monopolar or a bipolar probe,
device wherein the generator includes means for detecting the presence of a probe connected to the connector, comprising
- means (24,26) for producing monopolar or bipolar stimulation pulses selectively with or without disconnection of the connection (S₁, S₂) to the reference potential ;
- means for picking up at least one pulse signal produced by the variation of the potential induced on one and/or the other terminals (RING, TIP) and/or on the metallic housing (CASE) through application of said stimulation pulses ;
- discriminator means (28) adapted to analyze a shape characteristic of said at least one pulse signal and to deliver an indicator representative of the presence or the absence of a probe ; and
- control means for modifying at least one operating parameter of the device, selectively as a function of the indicator delivered by the discriminator means,
**characterized in that** said shape characteristic is the width of said at least one pulse signal.

2. The device according to claim 1, wherein the means for producing stimulation pulses are means adapted to apply a stimulation pulse with disconnection of the connection to the reference potential, and the discriminator means are means adapted to compare to a given threshold (Wₚ) the width of the pulse signal picked up on the terminal of the proximal electrode (RING) and on the housing (CASE).

3. The device according to claim 2, wherein the discriminator means are means adapted to deliver an indicator representative of the absence of a probe (figure 2) or of the non-implantation of the housing after connection of a probe (figure 3) when the width of the pulse signal on the housing (CASE) in less than said threshold (Wₚ).

4. The device according to claim 2, wherein the discriminator means are means adapted to deliver an indicator representative of the presence of an implanted monopolar probe (figure 5) when the width of the pulse signal picked up on the housing (CASE) is more or equal to said threshold (Wₚ) and the width of the pulse signal picked up on the terminal of the proximal electrode (RING) is less than said threshold (Wₚ).

5. The device according to claim 2, wherein the discriminator means are means adapted to deliver an indicator representative of the presence of an implanted bipolar probe (figure 4) when the width of the pulse signal picked up on the housing (CASE) and on the proximal terminal (RING) is more or equal to said threshold (Wₚ).

6. The device according to claim 1, wherein the means for producing stimulation pulses are means adapted to apply a bipolar stimulation pulse without disconnection of the connection to the reference potential, and the discriminator means are means adapted to compare the width of the pulse signal picked up on the housing (CASE) to a low threshold (W_{MIN}) and a high threshold (Wₚ).

7. The device according to claim 6, wherein the discriminator means are means adapted to deliver an indicator representative of the presence of a bipolar probe (figure 7) when the width of the pulse signal on the housing (CASE) in less than said low threshold (W_{MIN}).

8. The device according to claim 6, wherein the discriminator means are means adapted to deliver an indicator representative of the absence of a probe (figure 6) when the width of the pulse signal on the housing (CASE) is comprised between said low threshold (W_{MIN}) and said high threshold (Wp).

9. The device according to claim 6, wherein the discriminator means are means adapted to deliver an indicator representative of the presence of a monopolar probe (figure 8) when the width of the pulse signal on the housing (CASE) is equal to said high threshold (Wp).

10. The device according to claim 1, wherein the means for producing stimulation pulses are means adapted to apply a monopolar stimulation pulse without disconnection of the connection to the reference potential, and the discriminator means are means adapted to compare the width of the pulse signal picked up on the terminal of the proximal electrode (RING) to a low threshold (W_{MIN}) and a high threshold (Wₚ).

11. The device according to claim 10, wherein the discriminator means are means adapted to deliver an indicator representative of the absence of a probe (figure 6) when the width of the pulse signal on the terminal of the proximal electrode (RING) is comprised between said low threshold (W_{MIN}) and said high threshold (Wₚ).

12. The device according to claim 10, wherein the discriminator means are means adapted to deliver an indicator representative of the presence of a bipolar probe when the width of the pulse signal on the terminal of the proximal electrode (RING) is less than said low threshold (W_{MIN}).

13. The device according to claim 10, wherein the discriminator means are means adapted to deliver an indicator representative of the presence of a monopolar probe when the width of the pulse signal on the terminal of the proximal electrode (RING) is equal to said high threshold (Wp).

14. The device according to claim 1, wherein the means for producing stimulation pulses are means adapted to apply a bipolar stimulation pulse between the terminal of the proximal electrode (RING) and the terminal of the distal electrode (TIP) without disconnection of the connection to the reference potential, and the discriminator means are means adapted to compare the width of the pulse signal picked up on the housing (CASE) to a high threshold (Wp) and a low threshold (W_{MIN}).

15. The device according to claim 14, wherein the discriminator means are means adapted to deliver an indicator representative of the absence of a probe or of the non-implantation of the housing after connecting the probe (figure 6) when the width of the pulse signal on the housing (CASE) is comprised between said two thresholds.

16. The device according to claim 15, wherein the discriminator means are means adapted to deliver an indicator representative of the presence of a bipolar probe (figure 7) when the width of the pulse signal on the housing is less to said low threshold (W_{MIN}).

17. The device according to claim 15, wherein the discriminator means are means adapted to deliver an indicator representative of the presence of a monopolar probe (figure 8) when the width of the pulse signal on the housing (CASE) is equal to said high threshold (Wp).

18. The device according to claim 1, wherein, from an initial condition of the device before implantation, in which that device temporarily operates in a reduced functionality mode, the control means are means adapted to switch the device into a fully functional mode after delivering of an indicator representative of the presence of a probe.

19. The device according to claim 18, wherein the control means are means adapted to furthermore perform, after delivering an indicator representative of the presence of a probe, at least one action selected from : activating the device detection analog circuits, activating the physiological or activity sensors, programming the stimulation type - monopolar or bipolar - as a function of the delivered indicator ; initializing the operating algorithms of the device ; resetting and activating the diagnosis counters ; storing the implantation date.

20. The device according to claim 1, wherein safety means are furthermore provided, wherein said means are adapted to switch the device into a safety mode with monopolar stimulation when the device operates in a bipolar stimulation mode and in the case of an indicator non representative of the presence of a bipolar probe.

21. The device according to claim 1, wherein said safety means are means adapted to allow - when the device receives from an external programmer a parametering command in a bipolar stimulation mode - activating of the bipolar stimulation mode only after the discriminator means have delivered an indicator representative of the presence of a bipolar probe.

## Patentansprüche

1. Eine aktive implantierbare medizinische Vorrichtung, insbesondere ein Herzschrittmacher, ein Defibrillator, ein Kardioverter und/oder eine "Multisite"-Einrichtung, die geeignet ist, dem Herz niederenergetische elektrische Impulse zum Behandeln Herzrhythmusstörungen zu liefern,
wobei diese Vorrichtung ein metallisches Gehäuse (10) umfasst, das ein Generator enthält, welches geeignet ist, Stimulationsimpulse im monopolaren bzw. bipolaren Modus zu erzeugen und mit einem mit wenigstens zwei Anschlussklemmen (RING, TIP) ausgestatteten Konnektorkopf verbunden ist, wobei obengenannte Anschlussklemmen geeignet sind, mit den Elektroden einer mit dem Konnektor verbundenen Detektions- und Stimulationssonde verbunden zu sein, wobei obengenannte Sonde eine monopolare bzw. eine bipolare Sonden sein kann,
wobei obengenannte Sonde Mittel zur Detektion der Anwesenheit einer mit dem Konnektor verbundenen Sonde einschliesst, umfassen :
- Mittel (24, 26) um monopolare bzw. bipolare Stimulationsimpulse selektiv mit oder ohne Ausschaltung der Verbindung (S1, S2) mit dem Bezugspotential zu erzeugen ;
- Mittel zum Auffangen wenigstens eines durch die auf einer und/oder der anderen Anschlussklemme (RING, TIP) und/oder auf dem metallischen Gehäuse (CASE) durch Anlegen obengenannter Stimulationsimpulse induzierte Potentialvariation erzeugten Impulsignals ;
- Diskriminatormittel (28), die geeignet sind, eine Formcharakteristik obengenanntes wenigstens einen Impulssignals zu analysieren und einen für die An-oder Abwesenheit einer Sonde repräsentativen Indikator zu liefern, und
- Steuerungsmittel um wenigstens einen Betriebsparameter der Vorrichtung selektiv in Abhängigkeit vom durch die Diskriminatormittel gelieferten Indikator zu verändern,
**dadurch gekennzeichnet, dass** obengenannte Formcharakteristik die Breite obengenanntes wenigstens einen Impulssignals ist.

2. Die Vorrichtung gemäss Anspruch 1, in welcher die Stimulationsimpulse erzeugenden Mittel Mittel sind, die geeignet sind, einen Stimulationsimpuls mit Ausschaltung der Verbindung mit dem Bezugspotential anzulegen, und die Diskriminatormittel Mittel sind, die geeignet sind, mit einer vorgegebenen Schwelle (Wₚ) die Breite des auf der Anschlussklemme der proximalen Elektrode (RING) und auf dem Gehäuse (CASE) aufgefangenen Impulssignals zu vergleichen.

3. Die Vorrichtung gemäss Anspruch 2, in welcher die Diskriminatormittel Mittel sind, die geeignet sind, einen für die Abwesenheit einer Sonde (Zeichnung 2) und für die Nicht-Implantation des Gehäuses nach dem Verbinden einer Sonde (Zeichnung 3) repräsentativen Indikator zu liefern, wenn die Breite des Impulssignals auf dem Gehäuse (CASE) kleiner ist als die Schwelle (Wₚ).

4. Die Vorrichtung gemäss Anspruch 3, in welcher die Diskriminatormittel Mittel sind, die geeignet sind, einen für die Anwesenheit einer implantierten monopolaren Sonde repräsentativen Indikator zu liefern, wenn die Breite des auf dem Gehäuse (CASE) aufgefangenen Impulssignals gleich oder grösser ist als obengenannte Schwelle (Wp) und wenn die Breite des auf der Anschlussklemme der proximalen Elektrode (RING) aufgefangenen Impulssignals kleiner ist als obengenannte Schwelle (Wp).

5. Die Vorrichtung gemäss Anspruch 2, in welcher die Diskriminatormittel Mittel sind, die geeignet sind, einen für die Anwesenheit einer bipolaren implantierten Sonde (Zeichnung 4) repräsentativen Indikator zu liefern, wenn die Breite des auf dem Gehäuse (CASE) und auf der proximalen Anschlussklemme (RING) aufgefangenen Impulssignals gleich oder grösser ist als obengenannte Schwelle (Wp).

6. Die Vorrichtung gemäss Anspruch 1, in welcher die Stimulationsimpulse erzeugenden Mittel Mittel sind, die geeignet sind, einen bipolaren Stimulationsimpuls ohne Ausschaltung der Verbindung mit dem Bezugspotential anzulegen, und die Diskriminatormittel Mittel sind, die geeignet sind, die Breite des auf dem Gehäuse (CASE) aufgefangenen Impulssignals mit einer unteren (W_{MIN}) und einer oberen (Wp) Schwelle zu vergleichen.

7. Die Vorrichtung gemäss Anspruch 6, in welcher die Diskriminatormittel Mittel sind, die geeignet sind, einen für die Anwesenheit einer bipolaren Sonde (Zeichnung 7) repräsentativen Indikator zu liefern, wenn die Breite des Impulssignals auf dem Gehäuse (CASE) kleiner ist als obengenannte untere Schwelle (W_{MIN}).

8. Die Vorrichtung gemäss Anspruch 6, in welcher die Diskriminatormittel Mittel sind, die geeignet sind, einen für die Abwesenheit einer Sonde (Zeichnung 6) repräsentativen Indikator zu liefern, wenn die Breite des Impulssignals auf dem Gehäuse (CASE) zwischen obengenannter unteren Schwelle (W_{MIN}) und obengenannter oberen Schwelle (Wₚ) liegt.

9. Die Vorrichtung gemäss Anspruch 6, in welcher die Diskriminatormittel Mittel sind, die geeignet sind, einen für die Anwesenheit einer monopolaren Sonde (Zeichnung 8) repräsentativen Indikator zu liefern, wenn die Breite des Impulssignals auf dem Gehäuse (CASE) gleich ist wie obengenannte obere Schwelle (Wp).

10. Die Vorrichtung gemäss Anspruch 1, in welcher die Stimulationsimpulse erzeugenden Mittel Mittel sind, die geeignet sind, einen monopolaren Stimulationsimpuls ohne Ausschalten der Verbindung mit dem Bezugspotential anzulegen, und die Diskriminatormittel Mittel sind, die geeignet sind, die Breite des auf der Anschlussklemme der proximalen Elektrode (RING) aufgefangenen Impulssignals mit einer unteren Schwelle (W_{MIN}) und einer oberen Schwelle (Wp) zu vergleichen.

11. Die Vorrichtung gemäss Anspruch 10, in welcher die Diskriminatormittel Mittel sind, die geeignet sind, einen für die Abwesenheit einer Sonde (Zeichnung 6) repräsentativen Indikator zu liefern, wenn die Breite des Impulssignals auf der Anschlussklemme der proximalen Elektrode (RING) zwischen obengenannter unteren (W_{MIN}) und obengenannter oberen (Wp) Schwell liegt.

12. Die Vorrichtung gemäss Anspruch 10, in welcher die Diskriminatormittel Mittel sind, die geeignet sind, einen für die Anwesenheit einer bipolaren Sonde repräsentativen Indikator zu liefern, wenn die Breite des Impulssignals auf der Anschlussklemme der proximalen Elektrode (RING) kleiner ist als obengenannte untere Schwelle (W_{MIN}).

13. Die Vorrichtung gemäss Anspruch 10, in welcher die Diskriminatormittel Mittel sind, die geeignet sind, einen für die Anwesenheit einer monopolaren Sonde repräsentativen Indikator zu liefern, wenn die Breite des Impulssignals auf der Anschlussklemme der proximalen Elektrode (RING) gleich ist wie obengenannte obere Schwelle (Wₚ).

14. Die Vorrichtung gemäss Anspruch 1, in welcher die Stimulationsimpulse erzeugenden Mittel Mittel sind, die geeignet sind, einen bipolaren Stimulationsimpuls zwischen der Anschlussklemme der proximalen Elektrode (RING) und der distalen Elektrode (TIP) ohne Ausschalten der Verbindung mit dem Bezugspotential anzulegen, und die Stimulationsimpulse erzeugenden Mittel Mittel sind, die geeignet sind, die Breite des auf dem Gehäuse (CASE) aufgefangenen Impulssignals mit einer oberen (Wp) und einer unteren (W_{MIN}) Schwelle zu vergleichen.

15. Die Vorrichtung gemäss Anspruch 14, in welcher die Diskriminatormittel Mittel sind, die geeignet sind, einen für die Abwesenheit einer Sonde und für die Nicht-Implantation des Gehäuses nach dem Verbinden einer Sonde (Zeichnung 6) repräsentativen Indikator zu liefern, wenn die Breite des Impulssignals auf dem Gehäuse (CASE) zwischen jenen zwei Schwellen liegt.

16. Die Vorrichtung gemäss Anspruch 15, in welcher die Diskriminatormittel Mittel sind, die geeignet sind, einen für die Anwesenheit einer bipolaren Sonde (Zeichnung 7) repräsentativen Indikator zu liefern, wenn die Breite des Impulssignals auf dem Gehäuse (CASE) kleiner ist als obengenannte untere Schwelle (W_{MIN}).

17. Vorrichtung gemäss Anspruch 15, in welcher die Diskriminatormittel Mittel sind, die geeignet sind, einen für die Anwesenheit einer monopolaren Sonde (Zeichnung 8) repräsentativen Indikator zu liefern, wenn die Breite des Impulssignals auf dem Gehäuse (CASE) gleich ist wie obengenannte obere Schwelle (Wp).

18. Die Vorrichtung gemäss Anspruch 1, in welcher, ab einem Anfangszustand der Vorrichtung vor einer Implantation, wo diese Vorrichtung vorübergehend in einem Modus mit reduzierter Funktionalität arbeitet, die Steuerungsmittel Mittel sind, die geeignet sind, nach der Lieferung eines für die Anwesenheit einer Sonde repräsentativen Indikators die Vorrichtung in einen vollständig funktionellen Modus umzuschalten.

19. Die Vorrichtung gemäss Anspruch 18, in welcher die Steuerungsmittel Mittel sind, die geeignet sind, nach der Lieferung eines für die Anwesenheit einer Sonde repräsentativen Indikators ausserdem wenigstens eine Tätigkeit unter folgenden auszuführen : Aktivierung der analogen Detektionsschaltungen der Vorrichtung Aktivierung der physiologischen bzw. Aktivitätssensore ; Programmierung des Stimulationstyps, d.h. monopolaren oder bipolaren, in Abhängigkeit vom gelieferten Indikator, Initialisierung des Betriebsalgorithmus der Vorrichtung Zurücksetzung und Aktivierung der Diagnosezähler ; Speicherung des Implantationsdatums.

20. Die Vorrichtung gemäss Anspruch 1, in welcher Sicherheitsmittel vorgesehen sind, die geeignet sind, wenn die Vorrichtung mit einer bipolaren Stimulation auf einer bipolaren Sonde arbeitet und im Fall einer Lieferung eines für die Anwesenheit einer bipolaren Sonde nicht-repräsentativen Indikators die Vorrichtung in einen Sicherheitsmodus mit monopolaren Stimulation umzuschalten.

21. Die Vorrichtung gemäss Anspruch 1, in welcher obengenannte Sicherheitsmittel Mittel sind, die geeignet sind, wenn die Vorrichtung von einem externen Programmator einen Parametrierungsbefehl in einem bipolaren Stimulationsmodus bekommt, die Aktivierung des bipolaren Stimulationsmodus erst dann zu erlauben, wenn durch Diskriminatormittel ein für die Anwesenheit einer bipolaren Sonde repräsentativer Indikator geliefert wird.
